# EUROPÄISCHE PATENTSCHRIFT

(11) **EP 0 639 403 B1**
(45) Veröffentlichungstag und Bekanntmachung des Hinweises auf die Patenterteilung: **30.07.1997**
(21) Anmeldenummer: 94109267.8
(22) Anmeldetag: 16.06.1994
(51) Int. Cl.: B01J 23/46, C07C 209/68

(54) **Katalysator zur Herstellung von Bis-Para-aminocyclohexylmethan mit niedrigem Anteil an trans-trans-Isomer durch Hydrierung von Methylendianilin**
Catalyst for the production of bis-para-aminocyclohexylmethane with reduced trans-trans-isomer content by hydrogenation of methylenedianiline
Catalyseur pour la préparation de bis-para-aminocyclohexylméthane avec peu d'isomères trans-trans par hydrogénation de méthylènedianiline

(30) Priorität: 20.08.1993 DE 4328007
(43) Veröffentlichungstag der Anmeldung: 22.02.1995
(73) Patentinhaber: HÜLS AKTIENGESELLSCHAFT, 45764 Marl (DE)
(72) Erfinder: Maschmeyer, Dietrich, Dr., D-45772 Marl (DE); Thelen, Gerhard, Dr., D-48301 Nottuln (DE)

(56) Entgegenhaltungen:
- EP-A- 0 121 735
- EP-A- 0 231 788
- EP-A- 0 630 882
- FR-A- 2 354 815
- FR-A- 2 432 479
- US-A- 3 347 917

## Beschreibung

Die Erfindung betrifft einen Ruthenium- oder Rhodium-Katalysator zur Hydrierung von Methylendianilin (MDA) zu Bis-Para-aminocyclohexylmethan (PACM), wobei der Katalysator auf einem Träger aus einer speziellen Übergangstonerde aufgebracht ist.

Die Hydrierung von Methylendianilin wurde um 1947 entwickelt und um 1965 in den technischen Maßstab überführt. Die meisten Veröffentlichungen beschreiben Verfahren, die in Suspension unter Zusatz eines weitgehend unreaktiven Lösemittels arbeiten. Das Hydrierprodukt PACM fand nach und nach Eingang in immer mehr Anwendungen, bei denen die Isomerenverhältnisse sehr wichtig sind.

Die US-PS 2 511 028 und US-PS 2 606 925 beschreiben allgemein die Herstellung von PACM durch Hydrierung von MDA an einem Ruthenium-Katalysator. US-PS 2 606 928 zeigt die Herstellung von PACM mit erhöhtem cis-Anteil, als Katalysator dient Ruthenium mit Alkalizusatz auf Aktivkohle. Eine Weiterentwicklung stellt US-PS 3 697 449 dar, wobei der mit Alkali moderierte Katalysator auf Trägern aus Aluminiumoxid, Bariumsulfat oder Kieselgur in pulveriger Form eingesetzt wird; nach US-PS 3 636 108 wird der alkalimoderierte Katalysator zusätzlich mit Alkaliamid oder -methylat versetzt.

In der US-PS 3 636 108 und US-PS 3 644 522 wird beschrieben, wie während der Hydrierung oder anschließend der Gehalt an trans-trans-Isomer verschoben werden kann. Als Katalysator wird Ruthenium, gegebenenfalls mit Alkali modifiziert, auf einem schwach alkalisch oder amphoter reagierenden Träger, wie Kaliumcarbonat oder Erdalkalioxid, verwendet. Als weitere Schutzrechte hinsichtlich Ruthenium-Katalysatoren sind die US-PS 4 448 995 und US-PS 4 394 298 sowie DE-A 40 28 270, DE-A 27 45 172 und DE-A 25 20 848 zu nennen.

Gemäß US-PS 3 591 635, US-PS 3 856 862 und EP-A 0 662 212 und EP-A 0 392 435 werden für die Hydrierung Rhodium-Katalysatoren eingesetzt.

Schließlich beschreibt EP-PS 0 324 190 ein Verfahren zur Hydrierung von MDA im Festbett, die Zugabe von Lösemittel ist lediglich optional. Durch Einsatz eines Ruthenium-Katalysators (0,1 - 5 % Ruthenium) auf Tonerde mit relativ dick getränkter Randzone (mindestens 50 µm), einer BET-Oberfläche von 70 bis 280 m²/g und einem mittleren Porendurchmesser von 1,0 bis 32,0 nm kann eine hohe Aktivität und ein niedriger Anteil an trans-trans-Isomer erzielt werden. Vorzugsweise wird in lösemittelfreier Schmelze gearbeitet.

Aus den vorgenannten Literaturstellen wird evident, daß die Hydrierung von substituierten Anilinen, insbesondere MDA, zu Produkten mit hohem Anteil an cis- bzw. cis-cis-Isomer, besonders hinsichtlich Findung eines Katalysators, ein großes Problem darstellt. Dies trifft insbesondere auf die Durchführung der Reaktion im Festbett zu und dürfte Ursache für die geringe Zahl an Erfolgen in diesem Bereich sein.

Es ist ferner ersichtlich, daß das Vorurteil vorliegt, die Isomerenverteilung des Produktes nur durch Änderung der besonders genau einzuhaltenden Reaktionsbedingungen, eine Dotierung des Trägers mit Alkali- oder Erdalkaliverbindungen oder durch die Zugabe von Ammoniak während der Reaktion beeinflussen zu können. Den Maßnahmen der Alkalisierung und Zugabe von Ammoniak wird als Wirkung auch noch eine Zurückdrängung der Desamininierung und der Amingruppenisomerisierung zugeschrieben. Eine Absenkung der Reaktionstemperatur hat jedoch wegen der absinkenden Katalysatoraktivität technische Grenzen. Zum anderen macht es eine derartige Dotierung mit Alkalien wegen deren guter Löslichkeit offenbar erforderlich, die metallischen Aktivkomponenten des Katalysators in einer relativ großen Schichtdicke aufzubringen. Dadurch wird aber der An- und Abtransport der Reaktanten durch Diffusion sehr erschwert, was trotz der relativ langsam verlaufenden Reaktion zu einer Kontrolle von Reaktionsgeschwindigkeit und Selektivität durch die Geschwindigkeit des Stofftransportes und nicht durch die intrinsische Leistungsfähigkeit der Aktivkomponente führt.

Die Kanäle solcher tief imprägnierten Katalysatoren werden außerdem umso leichter durch hochviskose Phasen aus Hochsiedern, Oligomeren, Kohlenstoffablagerungen usw. blockiert, je kleiner mittlerer Porenradius und Porenvolumen sind. Außerdem ist für eine optimale Verteilung und Dispersion der Metallpartikel eine große BET-Oberfläche sowie ein großer mittlerer Porenradius erforderlich.

Das Verfahren gemäß EP-PS 0 324 190 zeigt bereits gute Aktivierungswerte und einen relativ geringen Anteil an trans-trans-Isomer. Für die mechanische Stabilität der dort verwendeten Tonerdeträger ist aber eine hohe Vorbehandlungstemperatur erforderlich, durch welche die Oberfläche dieser Materialien relativ schnell desaktiviert wird.

Des weiteren zeigten reaktionskinetische Untersuchungen, daß die Isomerenverteilung nicht nur ein Effekt der Acidität des Katalysators, sondern auch der Verweilzeit ist und mit Erhöhung derselben sich zu erhöhten trans-trans-Anteilen, d. h. in die unerwünschte Richtung, verschiebt, weil der Katalysator stärker isomerisierend wirkt. Mit einer zunehmenden Verlegung der inneren Poren eines dickschalig imprägnierten Katalysatorträgers sinken die Diffusionskoeffizienten für Edukte wie MDA und Produkte wie PACM relativ schnell, so daß nach relativ kurzer Laufzeit der Katalysatoren nicht nur eine Desaktivierung, sondern auch eine Verlängerung der Verweilzeit im Katalysatorinneren und damit eine Verschiebung der Selektivität in ungünstige Bereiche auftritt. Da diese nicht durch Temperaturerhöhung ausgeglichen werden kann, weil sich dadurch die Selektivität weiter in den nicht gewünschten Bereich verschieben würde, muß der Katalysator für derartige Prozesse in der Regel nach relativ kurzer Zeit ausgewechselt werden.

Es war daher Aufgabe der Erfindung, nach geeigneten Trägern für Katalysatoren auf Ruthenium- bzw. Rhodium-Basis zu suchen, die bei der Hydrierung von MDA zu PACM eine hohe Lebensdauer haben und zu Produkten mit niedrigem trans-trans-Gehalt führen.

Überraschend wurde nun gefunden, daß bestimmte Tonerden eine sehr gute Puffersubstanz für die Acidität darstellen, wenn Sorge getragen wird, daß die Oberfläche der Katalysatorträger chemisch noch aktiv genug und nicht durch thermische Behandlung desaktiviert ist. Dies ist der Fall, wenn sie in Form der Verbindungen Böhmit, Hydrargillit oder Bayerit, vorzugsweise Hydrargillit, vorliegt. Sie entfaltet dann eine Wirkung, die einer Alkalizugabe entspricht, jedoch mit dem Unterschied, daß Aluminium unter den Reaktionsbedingungen unlöslich ist, während Alkalikomponenten in den entsprechenden Reaktionsmedien beweglich sind und sich gegebenenfalls in bestimmten Zonen an- und abreichern können, wodurch es lokal zu Verschiebungen der gebildeten Isomerenverteilung in ungünstige Bereiche kommen kann. Tonerde ist jedoch im normalen pH-Bereich zwischen 4 und 10 sehr wenig löslich, so daß basisch puffernde Gruppen an der Oberfläche von Katalysatorträgern ortsfest erhalten bleiben.

Gegenstand der Erfindung sind daher Katalysatoren zur Herstellung von Bis-Para-aminocyclohexylmethan durch Hydrierung von Methylendianilin, welche dadurch gekennzeichnet sind, daß Ruthenium oder Rhodium in einer Menge von 0,05 bis 8 Gew.-%, vorzugsweise 0,2 bis 3 Gew.-% in einer Schichtdicke von 5 bis 150 µm, vorzugsweise 10 bis 80 µm, auf einen Träger aufgebracht wird, der aus einer kalzinierten und oberflächlich rehydratisierten Übergangstonerde besteht, wobei diese Tonerde in Suspension von 10 g in 300 ml Wasser bei 25 °C nach 60 min mindestens einen Gleichgewichts-pH-Wert von 8,2 und nach Zugabe von 10 ml 0,1 N Salzsäure zu dieser Suspension nach 30 Minuten mindestens noch einen pH-Wert von 6,0 ergibt.

Erforderliche Reaktivität und Puffervermögen der einzusetzenden Katalysatorträger werden also erfindungsgemäß durch Messung der Pufferung gegen Salzsäure in wäßriger Suspension bestimmt. Durch praktische Versuche hat sich ergeben, daß kalzinierte und oberflächlich rehydratisierte Übergangstonerden gemörsert, in Suspension von 10 g in 300 ml Wasser bei 25 °C mindestens einen Gleichgewichts-pH-Wert von 8,2 erzeugen sollten. Die gesamte Pufferkapazität wird so bestimmt, daß einer derartigen Suspension sukzessive Salzsäure zugegeben wird. Hinreichende Kapazität und Aktivität sind gegeben, wenn nach Zugabe von 10 ml 0,1 N Salzsäure zu dieser Suspension bei 25 °C nach 30 Minuten mindestens noch ein pH-Wert von 6,0 besteht.

Zusätzlich wurde gefunden, daß Katalysatorträger mit sehr rauher Oberfläche einen schnellen Stoffaustausch gestatten. Diese Rauhigkeit läßt sich am besten durch die Rauhtiefe beschreiben. Sie sollte zwischen 50 und 200 µm liegen.

Mit den Katalysatoren der Erfindung werden viele Nachteile der bisher verwendeten Systeme vermieden. Insbesondere kann, wenn eine ortsfeste Pufferkapazität mit sehr hoher Flächendichte vorliegt, wie dies insbesondere an böhmitischen und an hydrargillitischen Oberflächen der Fall ist, die Schichtdicke der Aktivkomponenten sehr stark verringert werden. Dadurch verkürzen sich die Diffusionswege unter Umständen so sehr, daß eine Verlegung der inneren Porosität des Trägers mit Hochsiedern usw. über lange Zeit keinen nennenswerten Einfluß auf die Reaktion mehr zu erlangen vermag. Die Zugänglichkeit der inneren Oberfläche kann darüber hinaus noch wesentlich verbessert werden, wenn man die Rauhigkeit der Trägeroberfläche möglichst groß macht.

Die erfindungsgemäßen Katalysatoren haben eine BET-Oberfläche von mindestens 70 m²/g und eine offene Porosität von mindestens 0,1 ml/g.

Ein weiterer Gegenstand der Erfindung ist ein Verfahren zur Herstellung der erfindungsgemäßen Katalysatoren, wobei man den Katalysatorträger unter Umwälzung bei einer Temperatur von mindestens 80 °C mit einer verdünnten Ruthenium-Nitrosylnitratlösung besprüht und danach trocknet. Anschließend erfolgt die Reduktion im Wasserstoffstrom mit einem Druck von mindestens 30 kPa (0,3 bar) bei Temperaturen zwischen 70 und 400 °C, vorzugsweise 140 bis 250 °C.

Der Einsatz von gleichwirkenden Stoffen anstelle von Tonerde, wie Oxiden und Hydroxiden des Eisens, Yttriums oder von Elementen der Lanthaniden sei eingeschlossen.

Tonerden, welche die gewünschte Pufferwirkung zunächst nicht zeigen, können auf dem Wege der Fällung oder durch eine über den Sol/Gel-Prozeß aufgebrachte Oberflächenbeschichtung mit Oxiden und Hydroxiden des Aluminiums, Eisens, Yttriums oder der Lanthaniden entsprechend modifiziert werden. Dabei soll eine BET-Oberfläche von mindestens 3m²/g, eine offene Porosität von mindestens 0,04 ml/g und eine Rauhtiefe von mindestens 50 µm erreicht werden.

Schließlich ist noch Gegenstand der Erfindung die Verwendung der erfindungsgemäßen Katalysatoren zur Hydrierung von Methylendianilin zu Bis-Para-aminocyclohexylmethan bei einer Temperatur von 140 bis 220°C und einem Druck von 6 bis 40 MPa (60 bis 400 bar).

### Beispiele:

### Beispiel 1: (Vergleich)

### Charakterisierung des Trägers

10 g Tonerdeträger SPHERALIT® SP 521 C von Procatalyse werden gemörsert und in 300 ml Wasser von 25 °C suspendiert. Zum Ausschluß der Pufferwirkung von Kohlensäure wird Stickstoff durch die Suspension geleitet. Es ergibt sich nach 60 min ein Gleichgewichts-pH-Wert von 6,7. Dann werden 10 ml 0,1 N Salzsäure zugegeben. Nach 60 min beträgt der pH-Wert der Suspension 4,3.

Der Träger liegt in Form von extrudierten Strängen mit 1,6 mm Durchmesser vor. Die Oberfläche ist sehr glatt und hat eine Rauhtiefe unter 20 µm.

### Herstellung und Testung eines Katalysators mit diesem Träger

Das Trägermaterial wird nun in einer rotierenden Trommel auf 90 bis 100 °C erhitzt und durch eine feine Düse mit einer 1 %igen Lösung von Rutheniumnitrosylnitrat in Wasser besprüht, bis 1 % Ruthenium auf den Träger aufgebracht ist. Das Material wird bei 200 °C getrocknet. Der so hergestellte Katalysator wird in einen Rohrreaktor von 1 m Länge eingebaut und bei 200 °C unter strömendem Wasserstoff von 100 kPa (1 bar) Druck reduziert. Anschließend wird eine Schmelze von MDA unter 30 MPa (300 bar) Wasserstoffdruck bei 150 °C in Rieselfahrweise über den Reaktor mit einer LHMV von 0,1 g MDA / (g Katalysator · h) gepumpt.

Nach 3 Betriebstagen ergab sich ein MDA-Umsatz von 41 % mit einem Restgehalt an MDA von 59 % und 17 % halbhydriertem Produkt; der Anteil an trans-trans-Isomer im voll hydrierten Produkt betrug 22 %.

Zur Verbesserung des Umsatzes wurde die Temperatur auf 180 °C erhöht. Dabei stieg der MDA-Umsatz auf 85 %, der Gehalt an halbhydriertem Material im Produkt betrug 20 %, und der trans-trans-Anteil im voll hydrierten Produkt 28,5 %.

### Beispiel 2 (erfindungsgemäß):

### Charakterisierung des Trägers:

10 g ALCOA® F-1 Tonerde werden gemörsert und in 300 ml Wasser von 25 °C suspendiert. Zur Vermeidung der Pufferung durch Kohlensäure wird wieder Stickstoff durchgeleitet. Es ergibt sich nach 60 min ein GleichgewichtspH-Wert von 9,2. Dann werden 10 ml 0,1 N Salzsäure zugegeben. Nach 60 min beträgt der pH-Wert der Suspension 7,3.

Der Träger liegt in Form von unregelmäßigen Bruchstücken von 1 bis 3 mm Größe vor. Die Oberfläche ist sehr rauh und hat eine Rauhtiefe von etwa 150 µm.

### Herstellung und Testung eines Katalysators mit diesem Träger

Das Trägermaterial wird in einer rotierenden Trommel auf 90 bis 100 °C erhitzt und durch eine feine Düse mit einer 1 %igen Lösung von Rutheniumnitrosylnitrat in Wasser besprüht, bis 1 % Ruthenium auf den Träger aufgebracht ist. Das Material wird bei 200 °C getrocknet. Der so hergestellte Katalysator wird in einen Rohrreaktor von 1 m Länge eingebaut und bei 200 °C unter strömendem Wasserstoff von 100 kPa (1 bar) Druck reduziert. Anschließend wird eine Schmelze von MDA unter 30 MPa (300 bar) Wasserstoffdruck bei 150 °C in Rieselfahrweise über den Reaktor mit einer LHMV von 0,1 g MDA / (g Katalysator · h) gepumpt.

Nach 3 Betriebstagen ergab sich ein MDA-Umsatz von über 99 % mit einem Restgehalt an MDA von 0,28 % sowie 4,9 % halbhydriertes Produkt; der Anteil an trans-trans-Isomer im voll hydrierten Produkt betrug 23,3 %. Zur Modifizierung des Umsatzes wurde die Temperatur auf 140 °C gesenkt. Dabei sank der MDA-Umsatz auf 97 %, der Gehalt an halbhydriertem Material im Produkt betrug 18 %, und der trans-trans-Anteil im voll hydrierten Produkt 20,5 %.

Damit ist gezeigt, daß bei wesentlich höherem Umsatz an MDA der Gehalt an trans- und insbesondere an trans-trans-Isomer auf dem gewünschten Niveau gehalten werden kann, daß also die Isomerisierungsaktivität des Katalysatorserheblich geringer ist.

### Beispiel 3: Langzeitverhalten / Vergleich

Der Katalysator gemäß Vergleichsbeispiel 1 wurde in einen Rohrreaktor von 1 m Länge eingebaut und bei 200 °C unter strömendem Wasserstoff von 100 kPa (1 bar) Druck reduziert.
Anschließend wurde eine Schmelze von MDA unter 30 MPa (300 bar) Wasserstoffdruck bei anfänglich 150 °C, später variierter Temperatur in Rieselfahrweise mit einer LHMV von 0,1 g MDA / (g Katalysator · h) über den Reaktor gefahren. Die Gehalte an MDA, halbhydriertem Produkt und Anteil an trans-trans-Isomer im PACM entwickelten sich im Produkt über die Laufzeit wie in Tabelle 1 angegeben.

**Tabelle 1**

| Laufzeit d | Temperatur °C | MDA % | halbhydr. % | trans-trans im PACM % |
|---|---|---|---|---|
| 3 | 150 | 58,6 | 17,2 | 22,6 |
| 5 | 150 | 64,7 | 12,5 | 22,7 |
| 9 | 150 | 54,8 | 16,9 | 20,6 |
| 10 | 160 | 49,9 | 16,9 | 19,9 |
| 11 | 180 | 15,0 | 56,9 | 28,3 |
| 12 | 180 | 15,2 | 21,1 | 26,7 |
| 13 | 180 | 17,3 | 29,7 | 27,1 |
| 14 | 180 | 19,1 | 33,6 | 25,9 |

Der Versuch zeigt, daß bei 150 °C nur sehr geringe Umsätze erzielt werden. Temperaturerhöhung erhöht die Umsätze, jedoch immer noch nicht in einen befriedigenden Bereich, gleichzeitig steigt jedoch schon der Gehalt an trans-trans-Isomer im Produkt von 20 bis auf 25,5 %. Mit längerer Verweilzeit bei 180 °C ist zu sehen, daß der Umsatz durch Alterung des Katalysators stark zurückgeht. Der Katalysator gemäß Vergleichsbeispiel 1 ist daher für den industriellen Einsatz wenig geeignet.

### Beispiel 4: Langzeitverhalten / erfindungsgemäß

Der Katalysator gemäß Beispiel 2 wurde in einen Rohrreaktor von 1 m Länge eingebaut und bei 200 °C unter strömendem Wasserstoff von 100 kPa (1 bar) Druck reduziert. Anschließend wurde eine Schmelze von MDA unter 30 MPa (300 bar) Wasserstoffdruck bei anfänglich 150 °C später variierter Temperatur in Rieselfahrweise mit einer LHMV von 0,1 g MDA / (g Katalysator · h) über den Reaktor gefahren. Die Gehalte an MDA, halbhydriertem Produkt und Anteil an trans-trans-Isomer im PACM entwickelten sich im Produkt über die Laufzeit wie in Tabelle 2 angegeben.

**Tabelle 2**

| Laufzeit d | Temperatur °C | MDA % | halbhydr. % | trans-trans im PACM % |
|---|---|---|---|---|
| 3 | 150 | 0,28 | 4,90 | 23,3 |
| 5 | 150 | 0,09 | 1,08 | 25,4 |
| 9 | 150 | 0,30 | 2,10 | 25,2 |
| 15 | 150 | 0,22 | 1,86 | 25,3 |
| 17 | 140 | 1,89 | 14,00 | 20,6 |
| 21 | 140 | 4,00 | 21,10 | 20,0 |
| 24 | 150 | 0,74 | 6,60 | 24,6 |
| 26 | 150 | 1,00 | 8,00 | 24,2 |

Der Versuch zeigt, daß bei 150 °C über lange Zeit sehr hohe Umsätze bei relativ niedrigem Gehalt an trans-trans-Isomer erzielt werden. Durch Absenkung der Temperatur läßt sich der Gehalt an trans-trans-Isomer bei nur leicht sinkenden Umsätzen steuern. Nach einer Laufzeit von 26 Tagen und einem vielfachen Durchsatz an Edukt gegenüber dem Vergleichsbeispiel 3 werden immer noch ausgezeichnete Umsätze erzielt, wie sie im Vergleichsbeispiel 3 überhaupt nicht annähernd erreicht werden. Das zeigt deutlich die in den besonderen Eigenschaften des Trägers und des Herstellverfahrens begründete Überlegenheit des erfindungsgemäßen Katalysators.

### Beispiel 5: (erfindungsgemäßer Rhodium-Katalysator)

ALCOA® F-1 Tonerde wird analog zu Beispiel 2 in der rotierenden Trommel bei 90 bis 100 °C durch eine feine Düse mit einer 1 %igen Rhodiumnitratlösung in Wasser besprüht, bis 1 % Rhodium auf den Träger aufgebracht ist. Das Material wird bei 200 °C getrocknet. Der so hergestellte Katalysator wird in einen Rohrreaktor von 1 m Länge eingebaut und bei 200 °C unter strömendem Wasserstoff von 100 kPa (1 bar) Druck reduziert. Anschließend wird eine Schmelze von MDA unter 30 MPa (300 bar) Wasserstoffdruck bei 150 °C in Rieselfahrweise mit einer LHMV von 0,1 g MDA / (g Katalysator · h) über den Reaktor gepumpt.

Nach 3 Betriebstagen ergab sich ein MDA-Umsatz von etwa 98,8 % mit einem Restgehalt an MDA von 1,2 % sowie 6,1 % halbhydriertem Produkt. Der Anteil an trans-trans-Isomer im voll hydrierten Produkt (PACM) betrug 24,4 %. Nach weiteren 6 Betriebstagen betrug der Umsatz noch 97,8 % bei unverändertem Gehalt an trans-trans-Isomer im Produkt.

Damit ist gezeigt, daß in derselben Weise Katalysatoren mit Rhodium hergestellt werden können, die in ihren Eigenschaften den vorher beschriebenen Rutheniumkatalysatoren gleichen.

## Patentansprüche

1. Katalysatoren zur Herstellung von Bis-Para-aminocyclohexylmethan durch Hydrierung von Methylendianilin,
dadurch gekennzeichnet,
daß Ruthenium oder Rhodium in einer Menge von 0,05 bis 8 Gew.-%, vorzugsweise 0,2 bis 3 Gew.-% in einer Schichtdicke von 5 bis 150 µm, vorzugsweise 10 bis 80 µm, auf einen Träger aufgebracht wird, der aus einer kalzinierten und oberflächlich rehydratisierten Übergangstonerde besteht, wobei diese unbeschichtete Tonerde in Suspension von 10 g in 300 ml Wasser bei 25 °C nach 60 Minuten mindestens einen Gleichgewichts-pH-Wert von 8,2 und nach Zugabe von 10 ml 0,1 N Salzsäure zu dieser Suspension nach 30 Minuten mindestens noch einen pH-Wert von 6,0 ergibt.

2. Katalysator gemäß Anspruch 1,
dadurch gekennzeichnet,
daß der Träger eine innere BET-Oberfläche von mindestens 70 m²/g und eine offene Porosität von mindestens 0,1 ml/g aufweist.

3. Katalysator gemäß den Ansprüchen 1 und 2,
dadurch gekennzeichnet,
daß die rehydratisierte Tonerdephase überwiegend aus Hydrargillit oder Bayerit besteht.

4. Katalysator gemäß den Ansprüchen 1 bis 3,
dadurch gekennzeichnet,
daß der Träger eine sehr rauhe Oberfläche mit Rauhtiefen zwischen 50 und 200 µm hat.

5. Katalysator gemäß Anspruch 1,
dadurch gekennzeichnet,
daß Tonerdeträger mit geringeren Gleichgewichts-pH-Werten zur Erreichung der erforderlichen Werte mit einer oberflächlichen Beschichtung von Oxiden und/oder Hydroxiden des Aluminiums, Eisens, Yttriums oder der Lanthaniden versehen werden.

6. Katalysator nach Anspruch 5,
dadurch gekennzeichnet,
daß der Träger eine BET-Oberfläche von mindestens 3 m²/g, eine offene Porosität von mindestens 0,04 ml/g und eine Rauhtiefe der Oberfläche von mindestens 50 µm aufweist.

7. Verfahren zur Herstellung eines Katalysators gemäß Anspruch 1,
dadurch gekennzeichnet,
daß der Träger unter Umwälzung bei einer Temperatur von mindestens 80 °C mit einer verdünnten Rutheniumnitrosylnitratlösung besprüht und anschließend getrocknet wird.

8. Verfahren gemäß Anspruch 7,
dadurch gekennzeichnet,
daß anschließend an die Trocknung noch eine Reduktion in Wasserstoff mit einem Druck von mindestens 30 kPa (0,3 bar) bei Temperaturen zwischen 70 und 400 °C, vorzugsweise zwischen 140 und 250 °C, vorgenommen wird.

9. Verwendung eines Katalysators gemäß den Ansprüchen 1 bis 6 zur Hydrierung von Methylendianilin zu Bis-Para-aminocyclohexylmethan bei einer Temperatur von 140 bis 220 °C und einem Druck von 6 bis 40 MPa (60 bis 400 bar).

## Claims

1. A catalyst for preparing bis-para-aminocyclohexylmethane by hydrogenation of methylenedianiline, characterized in that ruthenium or rhodium in an amount of from 0.05 to 8 % by weight, preferably from 0.2 to 3 % by weight, is applied with a layer thickness of from 5 to 150 µm, preferably from 10 to 80 µm, to a support comprising a calcined and surface-rehydrated transition alumina, a suspension of 10 g of this uncoated alumina in 300 ml of water at 25°C having an equilibrium pH of at least 8.2 after 60 minutes and still having a pH of at least 6.0 30 minutes after addition of 10 ml of 0.1 N hydrochloric acid to it.

2. A catalyst according to claim 1, characterized in that the support has an internal BET surface area of at least 70 m²/g and an open porosity of at least 0.1 ml/g.

3. A catalyst according to either of claims 1 and 2, characterized in that the rehydrated alumina phase comprises predominantly hydrargillite or bayerite.

4. A catalyst according to any of claims 1 to 3, characterized in that the support has a very rough surface with peak-to-valley heights of from 50 to 200 µm.

5. A catalyst according to claim 1, characterized in that alumina supports having lower equilibrium pH values are provided with a surface coating of oxides and/or hydroxides of aluminium, iron, yttrium or the lanthanides to achieve the required values.

6. A catalyst according to claim 5, characterized in that the support has a BET surface area of at least 3 m²/g, an open porosity of at least 0.04 ml/g and a peak-to-valley height of the surface of at least 50 µm.

7. A process for preparing a catalyst according to claim 1, characterized in that the support, while being circulated at a temperature of at least 80°C, is sprayed with a dilute ruthenium nitrosyl nitrate solution and is subsequently dried.

8. A process according to claim 7, characterized in that subsequent to drying a further reduction is carried out in hydrogen at a pressure of at least 30 kPa (0.3 bar) at temperatures from 70 to 400°C, preferably from 140 to 250°C.

9. Use of a catalyst according to any of claims 1 to 6 for hydrogenating methylenedianiline to bis-para-aminocyclohexylmethane at a temperature from 140 to 220°C and a pressure from 6 to 40 MPa (60 to 400 bar).

## Revendications

1. Catalyseur pour la fabrication de bis-para-aminocyclohexylméthane par hydrogénation de méthylèndianiline,
caractérisé en ce que
du ruthénium ou du rhodium sont appliqués sur un support en uns quantité de 0,05 à 8 % en poids, de préférence de 0,2 à 3 % en poids en une épaisseur de couche de 5 à 150 µm, de préférence de 10 à 80 µm, support qui se compose d'une alumine de transition calcinée et réhydraté superficiellement, tandis que cette alumine non mise en couche, en suspension de 1 g dans 300 ml d'eau à 25°C donne après 60 minutes au moins une valeur de pH d'équilibre de 8,2 et après addition de 10 ml d'acide chlorhydrique à 0,1 normal à cette suspension donne après 30 minutes au moins encore une valeur de pH de 6,0.

2. Catalyseur selon la revendication 1,
caractérisé en ce que
le support comporte une surface BET interne d'au moins 70 m²/g et une porosité ouverte d'au moins 0,1 ml/g.

3. Catalyseur selon les revendications 1 et 2,
caractérisé en ce que
la phase d'alumine réhydratée se compose principalement d'hydrargillite ou de bayérite.

4. Catalyseur selon les revendications 1 à 3.
caractérisé en ce que
le support a une surface très rugueuse avec des profondeurs de rugosité comprises entre 50 et 200 µm.

5. Catalyseur selon la revendication 1,
caractérisé en ce que
les supports d'alumine avec des valeurs de pH d'équilibre plus faibles sont munis pour atteindre les valeurs nécessaires d'une enduction d'oxydes et/ou d'hydroxydes d'aluminium, de fer, d'yttrium ou de lanthanides.

6. Catalyseur selon la revendication 5,
caractérisé en ce que
le support comporte une surface BET d'au moins 3 m²/g, une porosité ouverte d'au moins 0,04 ml/g et une profondeur de rugosité de la surface d'au moins 50 µm.

7. Procédé de fabrication d'un catalyseur selon la revendication 1,
caractérisé en ce que
le support est aspergé en rotation à une température d'au moins 80°C avec une solution de nitrosylnitrate de ruthénium étendue et ensuite est séché.

8. Procédé selon la revendication 7,
caractérisé en ce que
à la suite du séchage est prévue encore une réduction dans de l'hydrogène avec une pression d'au moins 30 Kpa (0,3 bar) à des températures comprises entre 70 et 400°C, de préférence entre 140 et 250°C.

9. Utilisation d'un catalyseur selon les revendications 1 à 6, destiné à l'hydrogénation de méthylèndianiline en bis-para-aminocyclohexylméthane à une température comprise entre 140 et 220°C et une pression de 6 à 40 Mpa (60 à 400 bars).
